# FASCICULE DE BREVET EUROPEEN

(11) **EP 0 954 373 B1**
(45) Date de publication et mention de la délivrance du brevet: **28.04.2004**
(21) Numéro de dépôt: 97953980.6
(22) Date de dépôt: 30.12.1997
(51) Int. Cl.: B01F 3/04, C02F 3/20

(54) **DISPOSITIF DE BRASSAGE ET D'AERATION D'UN LIQUIDE ET D'ELIMINATION DE LA MOUSSE DANS UNE CUVE DE TRAITEMENT DE CE LIQUIDE**
VORRICHTUNG ZUM RÜHREN UND BELÜFTEN EINER FLÜSSIGKEIT SOWIE ZUR SCHAUMENTFERNUNG IN EINEM FLÜSSIGKEITSBEHANDLUNGSBOTTICH
DEVICE FOR STIRRING AND AERATING A LIQUID AND ELIMINATING THE FOAM IN A VAT FOR TREATING THIS LIQUID

(30) Priorité: 08.01.1997 FR 9700116
(43) Date de publication de la demande: 10.11.1999
(73) Titulaire: Carbofil International, 44540 Saint-Mars la Jaille (FR)
(72) Inventeur: BOULANT, Alain, 44380 Pornichet (FR)
(74) Mandataire: Colas, Jean-Pierre
(86) Numéro de dépôt international: PCT/FR1997/002463
(87) Numéro de publication internationale: WO 1998/030319

(56) Documents cités:
- EP-A- 0 264 905
- EP-A- 0 687 497
- DE-U- 9 302 204

## Description

La présente invention a pour objet un dispositif de brassage et d'aération d'un liquide et d'élimination de la mousse dans une cuve de traitement de ce liquide, comme par exemple les déchets graisseux extraits des effluents urbains d'eaux usées ou des industries agro-alimentaires.

Comme on le sait, les effluents urbains d'eaux usées qui circulent dans les canalisations d'évacuation telles que les égouts, sont recueillis dans des stations d'épuration dans lesquelles sont effectuées diverses opérations de traitement afin de les épurer avant de les renvoyer dans le milieu naturel.

Cette épuration produit des sous-produits liés à l'épuration comme de la graisse, des boues....

Or, les décharges acceptent de moins en moins ce type de produits, de telle sorte que les stations d'épuration doivent être maintenant capables de les traiter pour les limiter au maximum.

Ainsi, les graisses piégées à l'entrée de la station d'épuration sont déversées dans une cuve de traitement, comme par exemple une cuve de dégradation biologique, qui comporte un agitateur mécanique permettant de brasser le liquide.

De plus, la cuve peut comporter intérieurement et à sa partie inférieure des rampes d'injection dans le liquide d'air sous pression permettant d'aérer et d'accentuer le brassage du liquide.

Mais, ce genre de dispositif d'aération et de brassage convient plus particulièrement pour des fluides peu chargés c'est à dire de faible viscosité et son rendement diminue rapidement pour des fluides chargés notamment en graisse.

De plus, lors du traitement du liquide comme par exemple les déchets graisseux, avec ce genre de dispositif, il se forme à la surface du liquide une couche importante de mousse qui s'envole et pollue l'environnement si bien qu'il est nécessaire de prévoir des moyens permettant d'éliminer cette mousse flottant à la surface du liquide lors du traitement de ce liquide.

Jusqu'à présent, ces moyens sont constitués par des agitateurs disposés au niveau de la surface du liquide et destinés à rabattre la mousse.

Mais, ces agitateurs ne donnent pas entièrement satisfaction et une partie de la mousse s'envole et pollue l'environnement.

L'invention a pour but d'éviter ces inconvénients en proposant un dispositif de brassage et d'aération d'un liquide comme par exemple des déchets graisseux extraits des effluents urbains d'eaux usées ou des industries agro-alimentaires qui permet, par des moyens simples et consommant peu d'énergie, de traiter de manière efficace ce genre de liquide et de récupérer la mousse flottant à la surface de ce liquide.

L'invention a donc pour objet un dispositif de brassage et d'aération d'un liquide et d'élimination de la mousse dans une cuve de traitement dudit liquide conforme aux revendications ci-annexées.

D'autres caractéristiques et avantages de l'invention apparaîtront au cours de la description qui va suivre, faite en référence aux dessins annexés, sur lesquels :
- la Fig. 1 est une vue schématique en coupe d'une cuve de traitement d'un liquide équipée d'un premier mode de réalisation d'un dispositif de brassage et d'aération dudit liquide, selon l'invention,
- la Fig. 2 est une vue en coupe selon la ligne 2-2 de la Fig. 1,
- la Fig. 3 est une vue schématique en coupe d'une cuve de traitement d'un liquide équipée d'un second mode de réalisation d'un dispositif de brassage et d'aération dudit liquide, selon l'invention,
- la Fig. 4 est une vue en coupe selon la ligne 4-4 de la Fig. 3,
- la Fig. 5 est une vue schématique partielle en coupe d'un troisième mode de réalisation d'un dispositif de brassage et d'aération du liquide, selon l'invention,
- la Fig. 6 est une vue schématique en coupe d'un accélérateur de liquide du dispositif de brassage et d'aération, selon l'invention,
- la Fig. 7 est une vue schématique en coupe des moyens d'aération de la surface du liquide,
- la Fig. 8 est une vue schématique partielle et en coupe d'un quatrième mode de réalisation d'un dispositif de brassage et d'aération du liquide, selon l'invention,
- la Fig. 9 est une vue schématique et à plus grande échelle de la pompe à vortex du quatrième mode de réalisation du dispositif, selon l'invention,
- la Fig. 10 est une vue en coupe selon la ligne 10-10 de la Fig. 9,
- la Fig. 11 est une vue schématique partielle et en coupe d'un cinquième mode de réalisation d'un dispositif de brassage et d'aération du liquide, selon l'invention,
- la Fig. 12 est une vue en coupe selon la ligne 12-12 de la Fig. 11,
- la Fig. 13 est une vue schématique partielle et en coupe d'un sixième mode de réalisation d'un dispositif de brassage et d'aération du liquide, selon l'invention, et
- la Fig. 14 est une vue en coupe selon la ligne 14-14 de la Fig. 13.

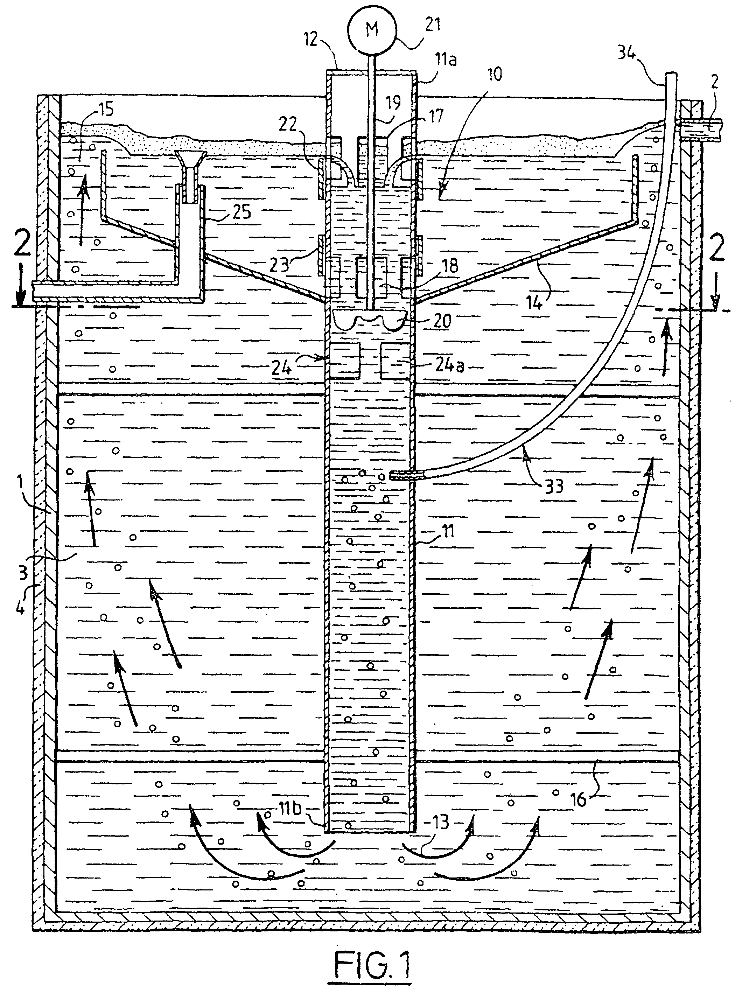

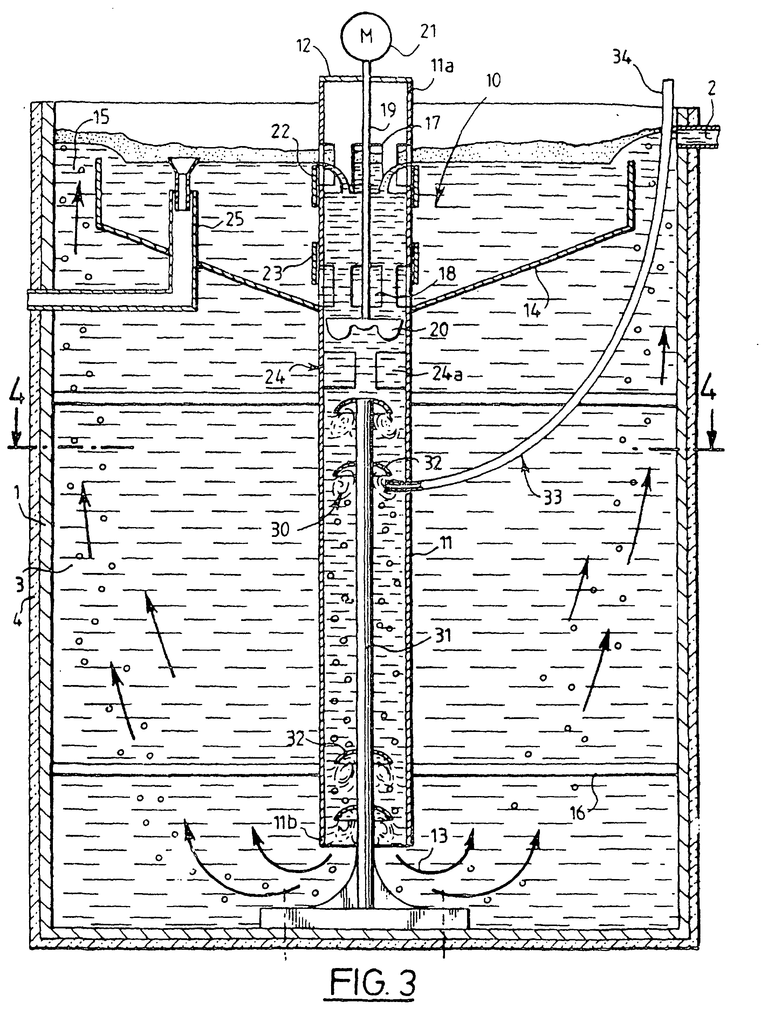

Sur les Figs. 1 et 3, on a représenté une cuve 1 munie à sa partie supérieure d'un orifice 2 d'entrée d'un liquide 3 à traiter, comme par exemple des effluents graisseux extraits des effluents urbains d'eaux usées.

La cuve de traitement 1 est recouverte sur sa face externe d'un isolant 4 et comporte un dispositif de brassage et d'aération du liquide et d'élimination de la mousse, désigné dans son ensemble par la référence 10.

Le dispositif 10 selon l'invention disposé à l'intérieur de la cuve 1 se compose d'une gaine verticale 11 s'étendant sur une partie de la hauteur de ladite cuve 1.

L'extrémité supérieure 11a de la gaine 11 est obturée par une plaque 12 et l'extrémité:.inférieure de cette gaine 11 est ouverte et ménage avec le fond de la cuve 1 un passage 13 pour le liquide 3.

Le dispositif 10 comporte également un entonnoir 14 évasé vers le haut dont le bord supérieur ménage avec la paroi de la cuve 1, un espace libre 15 pour le passage du liquide 3 et dont le bord inférieur est relié à la gaine 11 à une certaine distance de l'extrémité supérieure de ladite gaine 11.

L'ensemble constitué par la gaine 11 et l'entonnoir 14 est fixé à l'intérieur de la cuve 1 par des moyens appropriés, comme par exemple des pattes 16 qui ménagent entres elles des passages pour le liquide 3.

La gaine 11 comporte une première série de fenêtres 17 uniformément réparties sur le pourtour de cette gaine 11 et situées au niveau du liquide à l'intérieur de l'entonnoir 14.

Cette gaine 11 est également pourvue d'une seconde série de fenêtres 18 uniformément réparties sur le pourtour de ladite gaine 11 et situées au-dessus du fond de l'entonnoir 14.

Le dispositif 10 est aussi pourvu de moyens d'aspiration du liquide et de la mousse à l'intérieur de la gaine 11 par les fenêtres 17 et 18 et qui sont constitués par un arbre vertical 19 disposé à l'intérieur de cette gaine 11 et s'étendant de l'extrémité supérieure de ladite gaine 11 jusqu'au fond de l'entonnoir 14 et par une hélice principale 20 fixée à l'extrémité libre de l'arbre 19, au-dessous du fond dudit entonnoir 14.

Le diamètre de l'hélice principale 20 est légèrement inférieur au diamètre intérieur de la gaine 11.

L'arbre 19 et l'hélice 20 sont entraînés en rotation au moyen d'un moto-réducteur 21 qui est fixé sur la plaque 12 d'obturation de l'extrémité supérieure de ladite gaine 11.

Le dispositif 10 est pourvu de moyens de réglage du débit de liquide et de mousse aspiré par l'hélice principale 20 à l'intérieur de la gaine 11 par les fenêtres 17 et 18.

Ces moyens de réglage sont formés, d'une part, par un premier manchon 22 disposé autour de la gaine 11 au niveau de la première série de fenêtres 17 et par un second manchon 23 disposé autour de ladite gaine 11 au niveau de la seconde série de fenêtres 18.

La position de chaque manchon 22 et 23 par rapport respectivement aux fenêtres 17 et 18 est réglable verticalement par des moyens de serrage appropriés, non représentés.

Un organe anti-vortex 24 est disposé à l'intérieur de la gaine 11 au-dessous de l'hélice 20 pour éviter la rotation du liquide 3 dans ladite gaine 11.

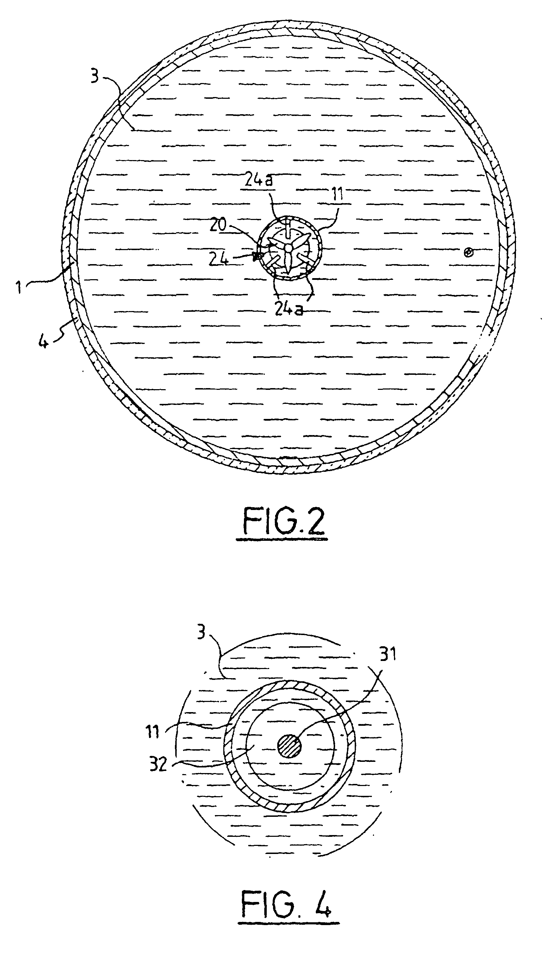

Ainsi que représenté à la Fig.2, cet organe anti-vortex 24 est constitué par trois plaques verticales 24a réparties à 120° à l'intérieur de ladite gaine 11.

Le dispositif 10 est encore pourvu de moyens de régulation du niveau de liquide 3 dans l'entonnoir 14 qui sont constitués par un tuyau de trop plein 25 disposé à l'intérieur dudit entonnoir 14 et dont la position est réglable verticalement.

Selon un second mode de réalisation représenté à la Fig. 3, le dispositif 10 comprend des moyens 30 de brassage statique par turbulence du liquide 3 à l'intérieur de la gaine 11 et disposés au-dessous de l'organe anti-vortex 24 dans ladite gaine 11.

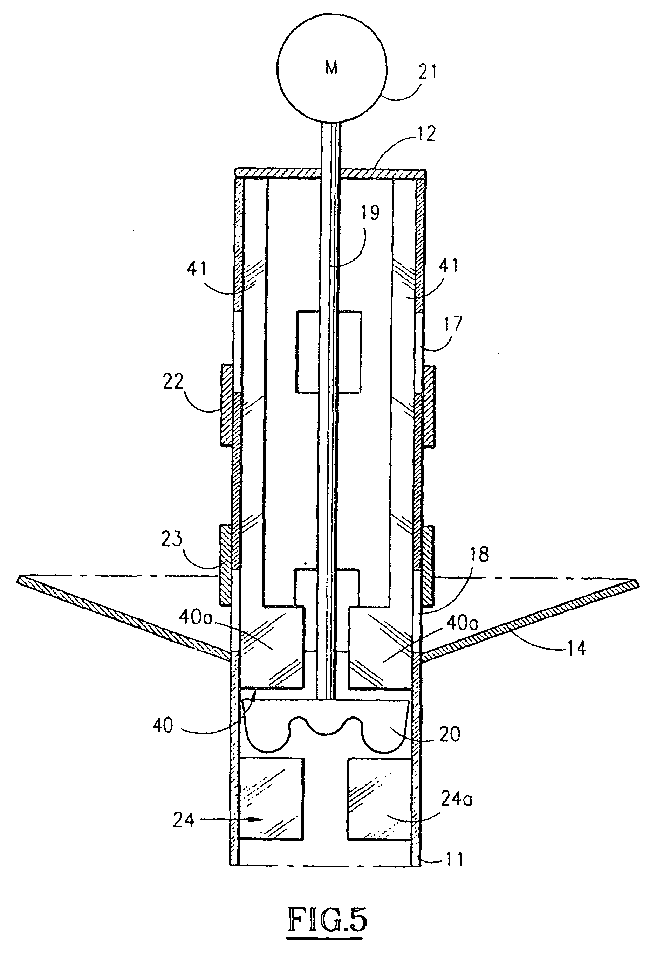

Comme représenté sur les Figs. 3 et 4, les moyens 30 de brassage statique par turbulence du liquide 3 sont formés; d'une part, par un tube vertical 31 disposé à l'intérieur de la gaine 11 et s'étendant du fond de la cuve 1 jusqu'au dessous de l'organe anti-vortex 24 et, d'autre part, par des obstacles statiques 32 superposés, disposés à l'intérieur de ladite gaine 11 et fixés sur le tube 31.

Le tube 31 est fixé sur le fond de la cuve 1 par des moyens appropriés.

Les obstacles statiques 32 ménagent avec la paroi interne de la gaine 11 des passages pour la circulation du liquide.

Ainsi que représenté à la Fig. 1, les obstacles statiques 32 sont répartis sur le tube 31, d'une part, au-dessous de l'organe anti-vortex 24 et, d'autre part, à la partie inférieure de la gaine 11.

Par exemple, le tube 31 comporte à sa partie supérieure, au-dessous de l'organe anti-vortex 24, deux obstacles statiques 32 superposés et à la partie inférieure de la gaine 11 également deux organes statiques 32 superposés.

Selon une variante, les obstacles statiques 32 peuvent être régulièrement répartis sur toute la portion du tube 31 disposée dans la gaine 11.

Selon un mode de réalisation représenté à la Fig. 3, les obstacles statiques 32 sont formés par des coupelles dont la concavité est dirigée vers le fond de la cuve 1.

Selon une variante les obstacles statiques 32 peuvent être formés par des disques.

Enfin, le dispositif 10 comporte des moyens 33 d'injection d'un gaz sous pression, comme par exemple de l'air, dans la gaine 11.

Ces moyens d'injection 33 sont formés par au moins une conduite 34 débouchant dans la gaine 11 par exemple à un niveau situé au-dessous des organes statiques 32 disposés à la partie supérieure du tube 31.

Le dispositif qui vient d'être décrit fonctionne de la manière suivante.

Le liquide 3 à traiter est déversé par l'orifice 2 dans la cuve 1 jusqu'à un niveau situé au-dessus du bord supérieur de l'entonnoir 14.

Ce liquide 3 se déverse également à l'intérieur de cet entonnoir 14.

Le moto-réducteur 21 entraîne en rotation l'arbre 19 qui lui même entraîne en rotation l'hélice principale 20.

Simultanément, le gaz sous pression est injecté dans la gaine 11 par l'intermédiaire des conduites 34.

La rotation de l'hélice principale 20 provoque une aspiration du liquide 3 dans la gaine 11 par les fenêtres 17 et 18 de telle sorte que ce liquide 3 circule en continu de haut en bas, dans cette gaine 11, puis passe dans l'espace 13 et circule de bas en haut entre la gaine 11 et la paroi interne de la cuve 1 et ainsi,de suite.

L'organe anti-vortex 24 empêche la rotation du liquide dans la gaine 11 au-dessous de l'hélice 20.

Le niveau de liquide 3 à l'intérieur de l'entonnoir 14 se trouve au-dessous du niveau de liquide à l'intérieur de la cuve 1 et ce niveau de liquide à l'intérieur de l'entonnoir 14 est réglé par le tuyau de trop plein 25 de telle sorte que ce niveau se trouve sensiblement à mi-hauteur des fenêtres 17.

Par ailleurs, du fait de l'aspiration créée par l'hélice 20 à l'intérieur de la gaine 11, le niveau de liquide à l'intérieur de cette gaine 11 se trouve au-dessous du niveau de liquide à l'intérieur de l'entonnoir 14.

L'air entraîné à l'intérieùr de la cheminée 11 par la rotation de l'hélice 20 ainsi que le gaz sous pression injecté dans ladite gaine 11 créent des bulles de gaz qui se dispersent dans le liquide 3 à l'intérieur de ladite gaine 11 sous l'effet de la rotation de l'hélice 20.

Lors de la circulation du liquide 3 à l'intérieur de la gaine 11, les obstacles statiques 32 créent, au-dessous de chacun de ceux-ci, un effet de turbulence, ce qui a pour effet de favoriser le brassage et l'aération du liquide 3.

Ces obstacles 32 présentent l'avantage d'être statiques, tout en favorisant le brassage et l'aération du liquide dans la gaine 11, et empêchent l'accrochage des filasses à l'intérieur de cette gaine 11.

Ainsi, le liquide 3 est brassé et les bulles de gaz se divisent en micro-bulles favorisant la dispersion du gaz et de ce fait l'aération du liquide 3.

Entre la paroi interne de la cuve 1 et la gaine 11, les bulles de gaz accélèrent la remontée du liquide favorisant la circulation de ce liquide à l'intérieur de la cuve 1 ce qui permet d'améliorer le rendement.

Ainsi, le liquide à traiter passe plusieurs fois dans la gaine 11 et subit donc plusieurs traitements d'oxygénation.

Le débit de liquide 3 peut être réglé en déplaçant les manchons 22 et 23 par rapport aux fenêtres 17 et 18.

Le brassage du liquide 3 par l'hélice 20 et les obstacles statiques 32 provoquent la formation de mousse à la surface du liquide 3 dans la cuve 1.

La rotation de cette hélice 20 à l'intérieur de la gaine 11 provoqué un pompage au niveau de la surface du liquide 3 à l'intérieur de l'entonnoir 14 et une aspiration de la mousse flottant à la surface du liquide, ainsi qu'une chute du niveau de ce liquide à l'intérieur de la gaine 11.

La mousse est donc entraînée, noyée et mélangée au liquide 3 à l'intérieur de la cuve 1 ce qui permet de l'éliminer progressivement.

La position des manchons 22 et 23 par rapport aux fenêtres 17 et 18 régule le débit de liquide et de mousse aspiré à l'intérieur de la gaine 11 par l'hélice 20.

Le choix de l'hélice principale 20 et la vitesse de rotation de cette hélice 20 permettent d'adapter la consommation d'énergie en fonction du liquide à traiter.

Dans le cas, où le dispositif selon l'invention ne comporte pas d'organes statiques 32 comme représenté sur la Fig. 1, des turbulences se créent naturellement à l'intérieur de la gaine 11.

Mais, le rendement est faible ce qui convient pour des liquides peu chargés.

Les obstacles statiques 32 favorisent le brassage du liquide par turbulence qui permet d'augmenter le rendement du dispositif.

Selon une variante, les moyens de brassage statique par turbulence sont formés par des obstacles statiques fixés sur la paroi interne de la gaine 11 et ménageant un passage axial pour la circulation du liquide 3.

Ces obstacles statiques sont régulièrement répartis sur toute la portion de la gaine 11 située au-dessous de l'organe anti-vortex 24 ou à l'intérieur de la gaine 1, d'une part, au-dessous de l'organe anti-vortex 24 et, d'autre part, à la partie inférieure de ladite gaine 11.

Dans ce cas également, les obstacles statiques sont formés par exemple par des coupelles dont la concavité est dirigée vers le fond de la cuve 1 ou par des disques.

Selon un troisième mode de réalisation représenté à la Fig.5, le dispositif comprend un second organe anti-vortex 40 disposé à l'intérieur de la gaine 11 et au-dessus de l'hélice principale 20.

Ce second organe anti-vortex 40 est formé par au moins deux plaques 40a verticales et prolongées chacune vers le haut par une nervure de renfort 41 s'étendant jusqu'à l'extrémité supérieure de ladite gaine 11.

De préférence, le second organe anti-vortex 40 est formé par quatre plaques 40a verticales uniformément réparties à l'intérieur de la gaine 11 et prolongées chacune vers le haut par une nervure de renfort 41.

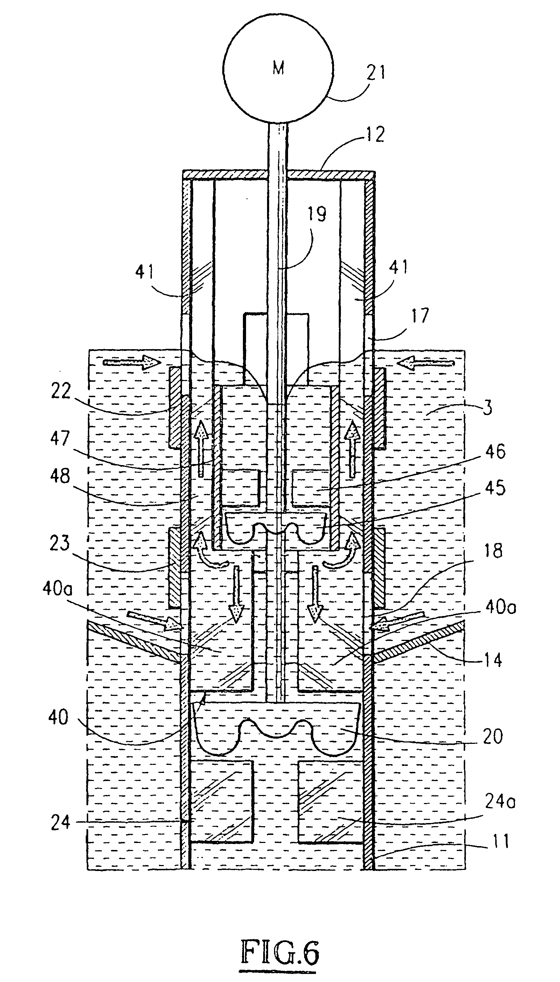

Selon un autre mode de réalisation représenté à la Fig. 6, le dispositif comprend un accélérateur de liquide à l'intérieur de la gaine 11.

Cet accélérateur de liquide est formé par une hélice secondaire 45 disposée au-dessus du second organe anti-vortex 40 et solidaire en rotation de l'arbre 19.

L'hélice secondaire 45 a un pas orienté dans le même sens que le pas de l'hélice principale 20.

De plus, l'accélérateur de liquide comprend également un organe anti-vortex 46 disposé au-dessus de l'hélice secondaire 45 et à l'intérieur d'une cheminée cylindrique 47.

La cheminée 47 est fixée sur le bord interne des nervures de renfort 41 de façon à ménager avec la gaine 11 et entre les nervures de renfort 41 des passages 48 pour le liquide.

L'hélice secondaire 45, l'organe anti-vortex 46 et la cheminée 47 sont situés entre les deux séries de fenêtres 17 et 18 qui sont, dans ce mode de réalisation pourvues des moyens de réglage du débit de liquide 3 et de mousse aspiré à l'intérieur de la gaine 11.

La rotation de l'hélice principale 20 provoque une aspiration du liquide 3 dans la gaine 11 par les fenêtres 17 et 18 de telle sorte que ce liquide 3 circule en continu de haut en bas dans cette gaine 11.

De plus, du fait de la rotation de l'hélice secondaire 45, l'aspiration du liquide 3 dans la gaine 11 par les fenêtres 17 et 18 est accélérée et une partie du liquide remonte par les passages 48 ménagés entre la gaine 11 et la cheminée 47 ce qui accentue encore ce phénomène d'accélération du liquidé à l'intérieur de ladite gaine 11.

Dans ce cas également, le réglage du niveau de liquide 3 à l'intérieur de la gaine 11 est toujours réalisé par la position des manchons 22 et 23 par rapport aux fenêtres 17 et 18.

L'organe anti-vortex 46 empêche la rotation du liquide dans la cheminée 47.

Ce mode de réalisation convient notamment pour des produits qui sont particulièrement moussants.

En effet, du fait de l'accélération de l'aspiration du liquide 3 à l'intérieur de la cheminée 47 et d'une recirculation d'une partie de ce liquide dans les passages 48, la mousse flottant à la surface du liquide 3 est aspirée à l'intérieur de ladite cheminée 47.

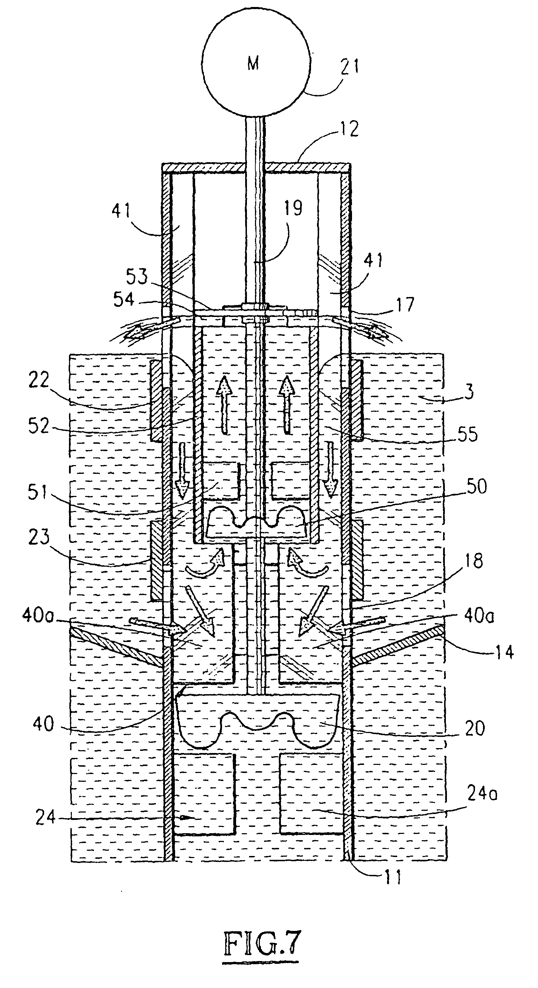

Selon encore un autre mode de réalisation représenté à la Fig. 7, le dispositif comporte des moyens d'aération de la surface du liquide 3 à l'intérieur de la cuve 1.

Ainsi que représenté sur cette figure, les moyens d'aération de la surface du liquide 3 sont formés par une hélice secondaire 50 disposée au-dessus du second organe anti-vortex 40 et solidaire en rotation de l'arbre 19.

Cette hélice secondaire 50 a un pas inversé par rapport au pas de l'hélice principale 20.

Les moyens d'aération de la surface du liquide 3 comprennent également un organe anti-vortex 51 disposé au-dessus de l'hélice secondaire 50 et à l'intérieur d'une cheminée cylindrique 52.

La cheminée 52 est fixée sur les bords internes des nervures de renfort 48 de façon à ménager avec la gaine 11 et lesdites nervures de renfort 48 des passages 55 pour le liquide 3.

L'hélice secondaire 50 et l'organe anti-vortex 51 sont situés entre les deux séries de fenêtres 17 et 18 et la cheminée 52 s'étend sensiblement de la partie médiane de la première série de fenêtres 17 jusqu'au bord supérieur de la seconde série de fenêtres 18.

Un couvercle 53 est monté sur l'arbre 19 au-dessus du bord supérieur de la cheminée 52.

La position verticale du couvercle 53 est réglable de façon à ménager avec le bord supérieur de la cheminée 52 un passage 54 réglable.

La rotation de l'hélice principale 20 provoque une aspiration du liquide 3, par les fenêtres 17 et 18 de telle sorte que ce liquide 3 circule de bas en haut dans la gaine 11.

Ainsi, une partie du liquide 3 est aspirée dans la gaine 11 par les fenêtres 18 et une autre partie du liquide 3 est aspirée par les fenêtres 17 et circule de haut en bas dans les passages 55 ménagés entre la cheminée 52 et la gaine 11.

De plus, compte tenu du pas de l'hélice secondaire 50 qui est inversé par rapport au pas de l'hélice principal 20, une partie du liquide est aspirée dans la cheminée 52 de bas en haut et est projetée dans l'air sur la surface du liquide 3 à l'intérieur de l'entonnoir 14 en passant par le passage 54.

Ainsi, la projection d'une partie du liquide permet d'aérer la surface du liquide 3 et de ce fait de diminuer la formation de mousse sur cette surface.

L'organe anti-vortex 51 empêche la rotation du liquide dans la cheminée 52 au-dessus de l'hélice secondaire 50.

Dans ce mode de réalisation également, le débit de liquide 3 dans la gaine 11 peut être réglé en déplaçant les manchons 22 et 23 par rapport aux fenêtres 17 et 18.

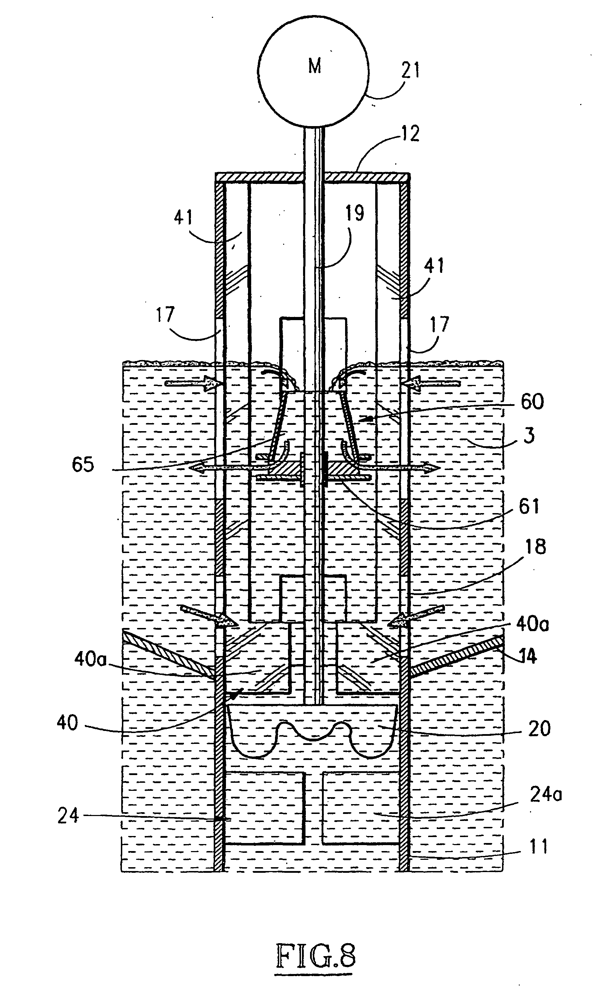

Selon un mode de réalisation représenté aux Figs. 8 à 10, le dispositif comprend une pompe à vortex 60 d'aspiration de la surface du liquide à l'intérieur de la gaine 11.

Cette pompe à vortex 60 est disposée au-dessus du second organe anti-vortex 40 et au niveau de la première série de fenêtres 17.

Cette pompe à vortex 60 est entraînée en rotation par l'arbre 19 et comprend, d'une part, un plateau circulaire 61 fixé sur cet arbre 19 et muni sur sa face supérieure de pales 62 radiales et verticales et, d'autre part, une cheminée 63 fixée sur le bord supérieur desdites pales 62.

L'ensemble constitué par le plateau 61, les pales 62 et la cheminée 63 est maintenu sur l'arbre 19 au moyen d'un manchon 61a dont la position verticale sur cet arbre 19 est réglable.

Le manchon 61a est fixé sur ledit arbre 19 au moyen d'un organe constitué par exemple par une vis de fixation, non représentée.

Comme représenté sur les Figs. 8 et 9, le bord supérieur des pales 62 est équipé d'une couronne 64 s'étendant vers l'extérieur de la cheminée 63.

Cette cheminée 63 s'étend vers le haut de la cuve 1 sensiblement de la partie médiane de la première série de fenêtres 17 jusqu'au bord supérieur des pales 62.

Cette cheminée 63 ménage un passage interne 65 qui communique, à sa partie inférieure, avec l'intérieur de la gaine 11 par des passages annulaires 66 ménagés entre les pales 62 et entre la couronne 64 et le plateau 61.

De préférence, la cheminée 63 est conique et la conicité est dirigée vers la partie supérieure de la cuve 1, comme représentée à la Fig. 9.

Selon une variante, la cheminée 63 peut être cylindrique.

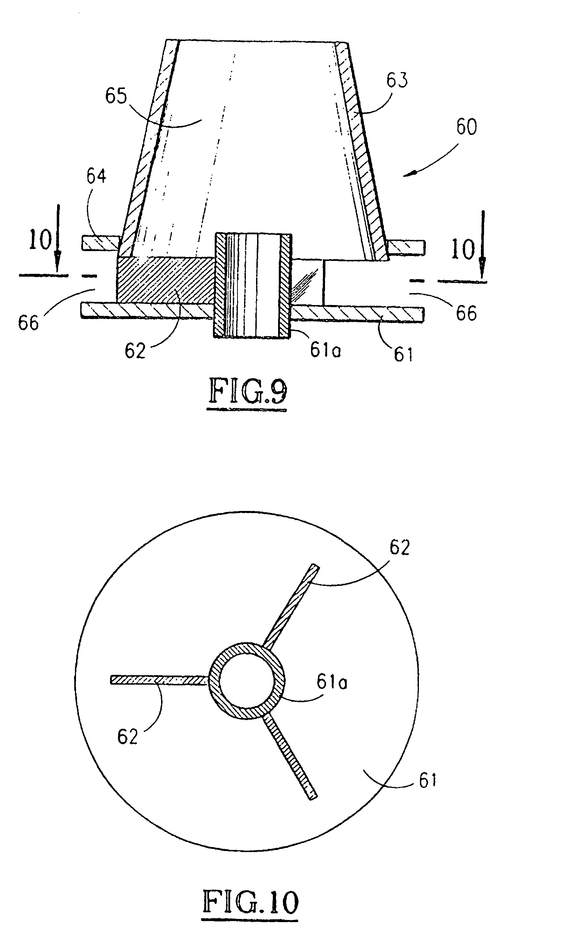

Les pales 62 sont planes ou incurvées et sont, de préférence, au nombre de trois, régulièrement réparties sur le plateau 61, ainsi que représenté à la Fig. 10.

La rotation de l'hélice principale 20 provoque une aspiration du liquide 3 dans la gaine 11 par les fenêtres 17 et 18 de telle sorte que ce liquide 3 circule en continu de haut en bas dans ladite gaine 11.

De plus, la rotation de la pompe à vortex 60 entraînée par l'arbre 19, c'est à dire la rotation de l'ensemble constitué par le plateau 61, les pales 62 et la cheminée 63 provoque une aspiration de la surface du liquide 3 dans le passage 65 de telle sorte que ce liquide passe ensuite par les passages annulaires 66 et soit réinjecté dans la cuve 1.

Dans ce mode de réalisation, le réglage du débit de liquide par les manchons 22 et 23 à l'intérieur de la gaine 11 n'est pas nécessaire.

Ce mode de réalisation convient particulièrement pour les produits très moussants du fait que la mousse flottant sur la surface du liquide 3 est aspirée par la pompe à vortex 60 ce qui permet d'éliminer rapidement et efficacement cette mousse.

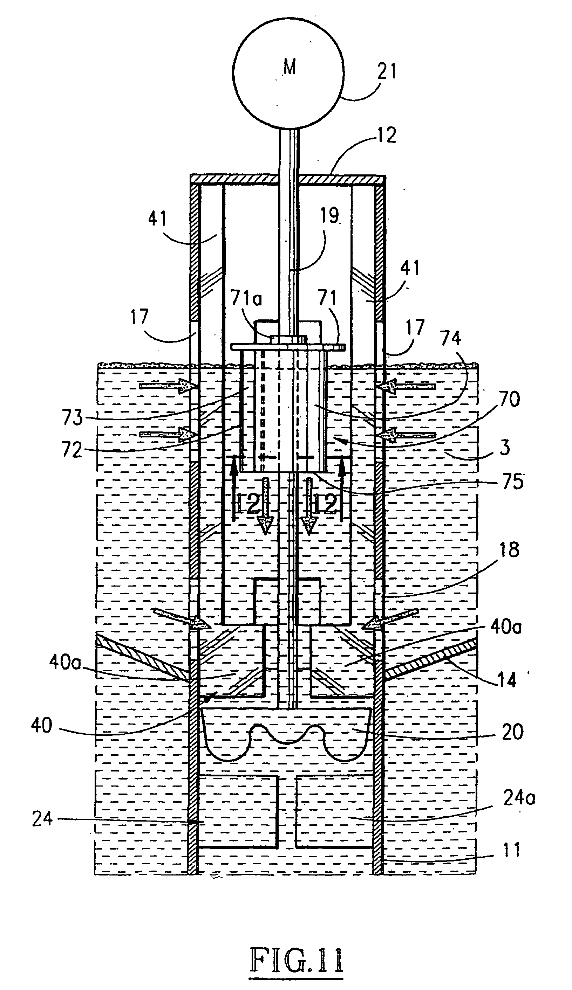

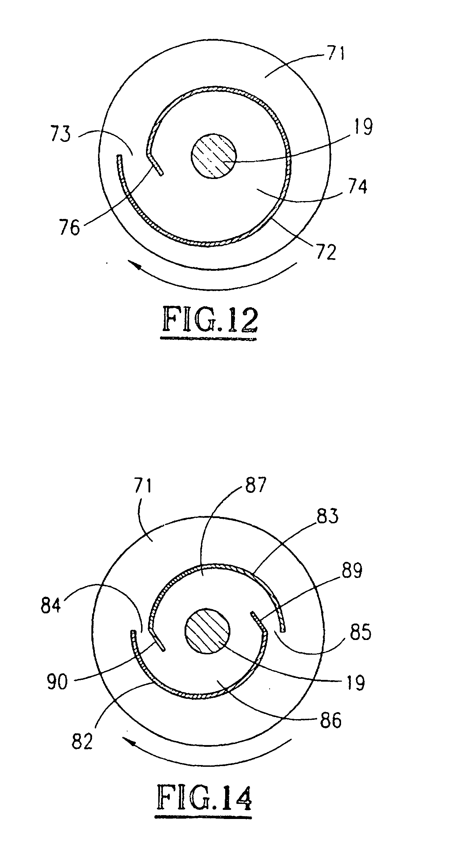

Selon un mode de réalisation représenté aux Figs. 11 et 12, le dispositif comprend une hélice monocanal 70 d'aspiration du liquide 3 à l'intérieur de la gaine 11.

Cette hélice monocanal 70 est disposée au-dessus du second organe anti-vortex 40 et au niveau de la première série de fenêtres 17.

L'hélice monocanal 70 est entraînée en rotation par l'arbre 19 et comprend, d'une part, un plateau circulaire 71 fixé sur cet arbre 19 et disposé au-dessus du niveau du liquide 3 dans la cuve 1 et, d'autre part, à l'intérieur du liquide 3, une plaque verticale 72 formant une spirale et fixée sur la face inférieure dudit plateau 71 autour de l'arbre 19.

Ainsi que représenté plus particulièrement à la Fig. 12, la plaque 72 délimite une entrée latérale 73 du liquide s'étendant sur toute la hauteur de ladite plaque 72, un canal vertical 74 de circulation de ce liquide de haut en bas et une sortie inférieure 75 dudit liquide.

L'ensemble constitué par le plateau 71 et la plaque verticale 72 est maintenu sur l'arbre 19 au moyen d'un manchon 71a dont la position verticale sur cet arbre 19 est réglable.

A cet effet, le manchon 71a est fixé sur ledit arbre 19 au moyen d'un organe constitué par exemple par une vis de fixation, non représentée.

Le bord interne de la plaque verticale 72 comporte un déflecteur vertical 76 incliné en direction du centre de la spirale formée par ladite plaque 72.

D'autre part, la distance séparant la plaque 72 et l'axe de l'arbre 19 diminue progressivement, ainsi que représenté à la Fig. 12.

La rotation de l'hélice monocanal 70 entraînée par l'arbre 19, c'est à dire la rotation de l'ensemble constitué par le plateau 71 et la plaque 72 dans le sens indiqué par la flèche sur la Fig.12, provoque une aspiration du liquide à l'intérieur de ladite plaque 72 par l'entrée latérale 73.

Ce liquide est brassé à l'intérieur du canal vertical 74 et circule dans ce canal 74 de haut en bas pour être évacué à l'intérieur de la gaine 11 par la sortie inférieure 75.

Le déflecteur 76 améliore le brassage du liquide à l'intérieur du canal vertical 74 et évite un retour du liquide par l'entrée latérale 73.

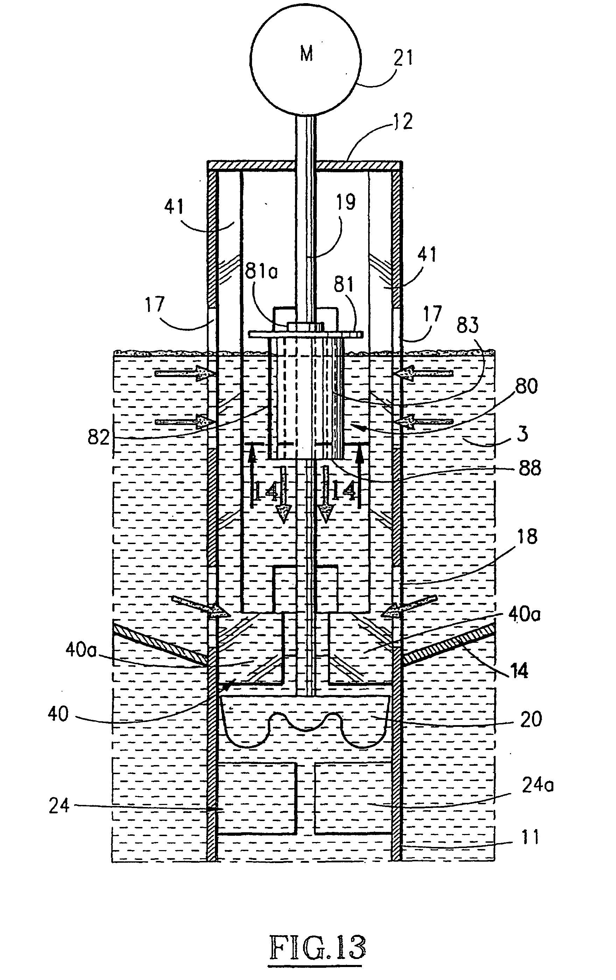

Selon un dernier mode de réalisation représenté sur les Figs. 13 et 14, le dispositif comprend une hélice bicanaux 80 d'aspiration du liquide 3 à l'intérieur de la gaine 11.

Cette hélice bicanaux 80 est disposée au-dessus du second organe anti-vortex 40 et au niveau de la première série de fenêtre 17.

L'hélice bicanaux 80 est entraînée en rotation par l'arbre 19 et comprend, d'une part, un plateau circulaire 81 fixé sur cet arbre 19 et disposé au-dessus du niveau du liquide 3 dans la cuve 1 et, d'autre part, à l'intérieur du liquide 3, deux plaques verticales 82 et 83.

Les plaques verticales 82 et 83 sont fixées sur la face inférieure du plateau 80 et ont chacune la forme de deux demi-coquilles qui sont opposées et décalées l'une par rapport à l'autre ainsi que représenté à la Fig.14.

Les deux plaques 82 et 83 délimitent deux entrées latérales 84 et 85 du liquide, deux canaux 86 et 87 de circulation de ces liquides de haut en bas et une sortie inférieure 88 dudit liquide.

L'ensemble constitué par le plateau 81 et les plaques verticales 82 et 83 est maintenu sur l'arbre 19 au moyen d'un manchon 81a dont la position verticale sur cet arbre 19 est réglable.

Le manchon 81a est fixé sur l'arbre 19 au moyen d'un organe constitué par exemple par une vis de fixation, non représentée.

Comme représenté plus particulièrement à la Fig. 14, le bord de chaque plaque 82 et 83 situé en regard du canal 86 ou 87 délimité par l'autre plaque comporte un déflecteur vertical, respectivement 89 et 90.

La rotation de l'hélice bicanaux 80 entraînée par la roue 19, c'est à dire la rotation de l'ensemble constitué par le plateau 81 et les deux plaques verticales 82 et 83 dans le sens indiqué par la flèche sur la Fig. 14 provoque une aspiration du liquide 3 par les fenêtres 17.

Ce liquide pénètre par les entrées latérales 84 et 85 et est brassé à l'intérieur des canaux 86 et 87 et circule de haut en bas à l'intérieur de ces canaux 86 et 87, puis est ensuite évacué dans la gaine 11 par la sortie inférieure 88.

Les déflecteurs 89 et 90 augmentent le brassage du liquide à l'intérieur des canaux 86 et 87 et évitent que ce liquide reflue par les entrées latérales 84 et 85.

Dans ces deux derniers modes de réalisation, le réglage du débit de liquide par les manchons 22 et 23 de la gaine 11 n'est pas nécessaire et ces deux derniers modes de réalisation sont plus particulièrement utilisés dans le cas où le niveau de liquide à l'intérieur de la cuve 1 est variable.

Pour améliorer le débit de l'hélice monocanal 70 ou de l'hélice bicanaux 80, une hélice de pompage, non représentée, peut être disposée sur l'arbre 19 au-dessous de l'hélice monocanal ou au-dessous de l'hélice bicanaux.

Le dispositif selon l'invention s'applique par exemple au traitement de l'eau, des effluents urbains, des eaux usées industrielles, des graisses par biodégradation, des matières de vidange, des déchets d'élevage et d'une manière générale à tous produits industriels biodégradables.

Le dispositif selon l'invention peut également s'appliquer par exemple à l'ozonation de l'eau par un gaz ozoné avec ou sans catalyseur, à la stabilisation des boues ou peut être utilisé en traitement thermophile ou pour des liquides chargés de matières en suspension par exemple organiques ou minérales.

Ce dispositif peut également être utilisé comme réacteur fonctionnant en lit fluidisé ou mobile.

Enfin, le dispositif selon l'invention présente l'avantage de traiter le liquide et simultanément d'éliminer progressivement la mousse flottant à la surface de ce liquide dans la cuve de traitement et ainsi d'éviter que cette mousse pollue l'environnement.

## Revendications

1. Dispositif de brassage et d'aération d'un liquide et d'élimination de la mousse dans une cuve (1) de traitement dudit liquide, comprenant à l'intérieur de la cuve (1) :
- une gaine (11) verticale s'étendant sur une partie de la hauteur de la cuve (1) et présentant, dans sa partie supérieure, au moins une première ouverture (17, 18) ménageant un passage d'entrée pour le liquide (3) prévu dans ladite cuve (1) lors de son utilisation et, au voisinage du fond de cette cuve, au moins une deuxième ouverture ménageant un passage de sortie (13) pour ledit liquide (3),
- un entonnoir (14) disposé autour de la partie supérieure de la gaine (11) et relié à ladite gaine (11), le bord supérieur de cet entonnoir (14) ménageant avec la paroi de la cuve (1) un espace libre (15) pour le passage dudit liquide (3),
- une hélice principale (20) située à l'intérieur de la gaine (11) au-dessous du fond de l'entonnoir (14),
- un arbre vertical (19) motorisé entraînant cette hélice (20) en rotation, s'étendant d'une part jusqu'au dessus de l'extrémité supérieure de la gaine (11) et d'autre part jusqu'en dessous du fond de l'entonnoir (14),
- un organe anti-vortex (24) disposé à l'intérieur de la gaine (11), au-dessous de ladite hélice (20),
- des moyens (33) d'injection d'un gaz sous pression dans la gaine (11), au-dessous de l'organe anti-vortex (24),
**caractérisé en ce qu'**il comprend en outre des moyens de brassage (31, 32) uniquement statiques par turbulence dudit liquide (3) à l'intérieur de la gaine (11), ces moyens étant disposés au-dessous de l'organe anti-vortex (24).

2. Dispositif selon la revendication 1, **caractérisé en ce qu'**il comprend :
- une première série de fenêtres (17) ménagées à la partie supérieure de la gaine (1) et situées de manière à se trouver au niveau du liquide (3) prévu dans ladite cuve (1) lors de son utilisation à l'intérieur de l'entonnoir (14),
- une seconde série de fenêtres (18) ménagées dans la gaine (11), directement au-dessus du fond de l'entonnoir (14), et
- des moyens (25) de régulation du niveau dudit liquide (3) dans l'entonnoir (14).

3. Dispositif selon la revendication 2, **caractérisé en ce qu'**il comprend des moyens (22,23) de réglage du débit du liquide (3) prévu dans ladite cuve (1) lors de son utilisation et de mousse aspiré par l'hélice (20) à l'intérieur de la gaine (11).

4. Dispositif selon la revendication 3, **caractérisé en ce que** les moyens de réglage du débit du liquide (3) prévu dans ladite cuve (1) lors de son utilisation et de mousse aspiré par ladite hélice (20) sont formés, d'une part, par un premier manchon (22) disposé autour de la gaine (11) au niveau de la première série de fenêtres (17) et, d'autre part, par un second manchon (23) disposé autour de ladite gaine (11) au niveau de la seconde série de fenêtres (18), la position desdits manchons (22, 23) par rapport aux fenêtres (17, 18) correspondantes étant réglable verticalement.

5. Dispositif selon l'une quelconque des revendications précédentes, **caractérisé en ce que** les moyens de brassage statiques par turbulence sont formés, d'une part, par un tube vertical (31) disposé à l'intérieur de la gaine (11) et s'étendant du fond de la cuve (1) jusqu'au dessous de l'organe anti-vortex (24) et, d'autre part, par des obstacles statiques (32) superposés, disposés à l'intérieur de ladite gaine (11) et fixés sur ledit tube (31).

6. Dispositif selon la revendication 5, **caractérisé en ce que** les obstacles statiques (32) ménagent avec la paroi interne de la gaine (11) des passages pour la circulation du liquide (3) prévu dans ladite cuve (1) lors de son utilisation.

7. Dispositif selon les revendications 5 et 6, **caractérisé en ce que** les obstacles statiques (32) sont régulièrement répartis sur toute la portion du tube (31) disposée dans la gaine (11).

8. Dispositif selon les revendications 5 et 6, **caractérisé en ce que** les obstacles statiques (32) sont répartis sur le tube (31), d'une part, au-dessous de l'organe anti-vortex (24) et, d'autre part, à la partie inférieure de la gaine (11).

9. Dispositif selon la revendication 5, **caractérisé en ce que** les moyens de brassage statiques par turbulence sont formés par des obstacles statiques fixés sur la paroi interne de la gaine (11) et ménageant un passage axial pour la circulation du liquide (3) prévu dans ladite cuve (1) lors de son utilisation.

10. Dispositif selon la revendication 9,. **caractérisé en ce que** les obstacles statiques sont régulièrement répartis sur toute la portion de la gaine (11) située au-dessous de l'organe anti-vortex (24).

11. Dispositif selon la revendication 10, **caractérisé en ce que** les obstacles statiques sont répartis à l'intérieur de la gaine (11), d'une part, au dessous de l'organe anti-vortex (24) et, d'autre part, à la partie inférieure de ladite gaine (11).

12. Dispositif selon l'une quelconque des revendications 5 à 11, **caractérisé en ce que** les obstacles statiques sont formés par des coupelles (32) dont la concavité est dirigée vers le fond de la cuve (1).

13. Dispositif selon l'une quelconque des revendications 5 à 11, **caractérisé en ce que** les obstacles statiques sont formés par des disques.

14. Dispositif selon l'une quelconque des revendications précédentes, **caractérisé en ce que** la cuve (1) est isolée.

15. Dispositif selon l'une quelconque des revendications précédentes lorsqu'elle dépend de la revendication 2, **caractérisé en ce que** les moyens de régulation du niveau dans l'entonnoir (14) du liquide (3) prévu dans ladite cuve (1) lors de son utilisation sont formés par un tuyau de trop plein (25) disposé à l'intérieur dudit entonnoir (14) et dont la position est réglable verticalement.

16. Dispositif selon l'une quelconque des revendications précédentes, **caractérisé en ce qu'**il comprend un second organe anti-vortex (40) disposé à l'intérieur de la gaine (11) et au-dessus de l'hélice principale (20), ce second organe anti-vortex (40) étant formé par au moins deux plaques (40a) verticales et prolongées chacune vers le haut par une nervure de renfort (41) s'étendant jusqu'à l'extrémité supérieure de ladite gaine (11).

17. Dispositif selon l'une quelconque des revendications précédentes, **caractérisé en ce qu'**il comprend un accélérateur (45, 46, 47) de liquide à l'intérieur de la gaine (11).

18. Dispositif selon l'une quelconque des revendications 2, 16 et 17, **caractérisé en ce que** l'accélérateur de liquide est formé par, d'une part, une hélice secondaire (45) disposée au-dessus du second organe anti-vortex (40) solidaire en rotation de l'arbre (19) et de pas orienté dans le même sens que le pas de l'hélice principale (20) et, d'autre part, un organe anti-vortex (46) disposé au-dessus de l'hélice secondaire (45) et à l'intérieur d'une cheminée cylindrique (47) fixée sur les nervures de renfort (41), l'hélice secondaire (45), l'organe anti-vortex (46) et la cheminée (47) étant situés entre les deux séries de fenêtres (17, 18).

19. Dispositif selon l'une quelconque des revendications 1 à 16, **caractérisé en ce qu'**il comprend des moyens (50, 51, 52) d'aération de la surface du liquide prévu dans ladite cuve (1) lors de son utilisation.

20. Dispositif selon les revendications 2, 16 et 19, **caractérisé en ce que** les moyens d'aération de la surface du liquide prévu dans ladite cuve (1) lors de son utilisation sont formés par, d'une part, une hélice secondaire (50) disposée au-dessus du second organe anti-vortex (40), solidaire en rotation de l'arbre (19) et de pas inversé par rapport au pas de l'hélice principale (20) et, d'autre part, un organe anti-vortex (51) disposé au-dessus de l'hélice secondaire (50) et à l'intérieur d'une cheminée cylindrique (52) fixée sur les nervures de renfort (41), l'hélice secondaire (50) et l'organe anti-vortex (51) étant situés entre les deux séries de fenêtres (17, 18) et la cheminée (52) s'étendant sensiblement de la partie médiane de la première série de fenêtres (17) jusqu'à presque le bord supérieur de la seconde série de fenêtres (18), un couvercle (53) étant monté sur l'arbre (19) au-dessus du bord supérieur de la cheminée (52) et ménageant avec ledit bord supérieur un passage (54) réglable.

21. Dispositif selon les revendications 2 et 16, **caractérisé en ce qu'**il comprend une pompe à vortex (60) d'aspiration à l'intérieur de la gaine (11) de la partie du liquide prévu dans ladite cuve (1) lors de son utilisation qui est située au voisinage de la surface de ce liquide, disposée au-dessus du second organe anti-vortex (40) et au niveau de la première série de fenêtres (17).

22. Dispositif selon la revendication 21, **caractérisé en ce que** la pompe à vortex (60) comprend, d'une part, un plateau circulaire (61) solidaire en rotation de l'arbre (19) et muni sur sa face supérieure de pales (62) radiales et verticales et, d'autre part, une cheminée (63) fixée sur le bord supérieur des pales (62) et s'étendant vers le haut de la cuve (1) sensiblement de la partie médiane de la première série de fenêtres (17) jusqu'audit bord supérieur des pales (62).

23. Dispositif selon la revendication 22, **caractérisé en ce que** la cheminée (63) est conique et dont la conicité est dirigée vers la partie supérieure de la cuve (1).

24. Dispositif selon la revendication 22, **caractérisé en ce que** la cheminée (63) est cylindrique.

25. Dispositif selon l'une quelconque des revendications 22 à 24, **caractérisé en ce que** les pales (62) sont planes ou incurvées.

26. Dispositif selon les revendications 2 et 16, **caractérisé en ce qu'**il comprend une hélice monocanal (70) d'aspiration à l'intérieur de la gaine (11) du liquide (3) prévu dans ladite cuve (1) lors de son utilisation, disposée au-dessus du second organe anti-vortex (40) et au niveau de la première série de fenêtres (17).

27. Dispositif selon la revendication 26, **caractérisé en ce que** l'hélice monocanal (70) comprend, d'une part, un plateau circulaire (71) solidaire en rotation de l'arbre (19) et disposé au-dessus du niveau devant être atteint par le liquide (3) prévu dans ladite cuve (1) lors de son utilisation et, d'autre part, s'étendant en partie au-dessous dudit niveau, une plaque verticale (72) formant une spirale et fixée sur la face inférieure dudit plateau (71) autour de l'arbre (19), ladite plaque (72) délimitant une entrée latérale (73) dudit liquide, un canal vertical (74) de circulation dudit liquide de haut en bas et une sortie inférieure (75) dudit liquide.

28. Dispositif selon la revendication 27, **caractérisé en ce que** le bord interne de la plaque verticale (72) comporte un déflecteur vertical (76) incliné en direction du centre de la spirale.

29. Dispositif selon les revendications 2 et 16, **caractérisé en ce qu'**il comprend une hélice bicanaux (80) d'aspiration à l'intérieur de la gaine (11) du liquide (3) prévu dans ladite cuve (1) lors de son. utilisation, disposée au-dessus du second organe anti-vortex (40) et au niveau de la première série de fenêtres (17).

30. Dispositif selon la revendication 29, **caractérisé en ce que** l'hélice bicanaux (80) comprend, d'une part, un plateau circulaire (81) solidaire en rotation de l'arbre (19) et disposé au-dessus du niveau devant être atteint par le liquide (3) prévu dans ladite cuve (1) lors de son utilisation et, d'autre part, s'étendant en partie sous ledit niveau, deux plaques verticales (82, 83) fixées sur la face inférieure dudit plateau (81) et ayant la forme de deux demi-coquilles opposées et décalées l'une part rapport à l'autre, lesdites plaques (82, 83) délimitant deux entrées latérales (84, 85) du liquide, deux canaux verticaux (86, 87) de circulation de ce liquide de haut en bas et une sortie inférieure (88) dudit liquide.

31. Dispositif selon la revendication 30, **caractérisé en ce que** le bord de chaque plaque (82, 83) situé en regard du canal (86, 87) délimité par l'autre plaque comporte un déflecteur vertical (89, 90).

32. Dispositif selon l'une quelconque des revendications 26 à 31, **caractérisé en ce qu'**une hélice de pompage est fixée sur l'arbre (19) au-dessous de l'hélice monocanal (70) ou de l'hélice bicanaux (80).

## Claims

1. Device for stirring and aerating a liquid and eliminating the foam in a tank (1) for treating said liquid, comprising inside the tank (1):
- a vertical sheath (11) extending in a part of the height of the tank (1) and comprising, in its upper part, at least a first opening (17, 18) providing an entry passage for the liquid (3) during operation of said tank (1) and, near the bottom of this tank, at least a second opening providing an exit passage (13) for the liquid (3),
- a funnel (14) disposed around the upper part of the sheath (11) and connected to said sheath (11), the upper edge of said funnel (14) defining with the wall of the tank (1) a free space (15) for the passage of the liquid (3) ,
- a main propeller (20) disposed inside the sheath (11) below the bottom of the funnel (14),
- a vertical shaft (19) driven by an engine, said shaft driving said propeller (20) in rotation and extending, on one hand, up to the upper end of the sheath (11) and, on the other hand, down to below the bottom of the sheath (14),
- an anti-vortex means (24) disposed inside the sheath (11), below said propeller (20),
- means (33) for injecting a gas under pressure in the sheath (11), below the anti-vortex means (24),
**characterized in that** it also comprises uniquely static stirring means (31, 32) for stirring said liquid (3) by turbulence inside the sheath (11), these means being disposed below the anti-vortex means (24).

2. Device according to claim 1, **characterized in that** it comprises:
- a first series of ports (17) provided in the upper part of the sheath (1) and situated so as to be at the level of the liquid (3) in the funnel (14) during operation of said tank (1),
- a second series of ports (18) provided in the sheath (11), directly above the bottom of the funnel (14), and
- means (25) for regulating the level of the liquid (3) in the funnel (14).

3. Device according to claim 2, **characterized in that** it comprises means (22, 23) for regulating the flow of liquid (3) during operation of said tank (1) and of the foam sucked by the propeller (20) into the sheath (11).

4. Device according to claim 3, **characterized in that** the means for regulating the flow of the liquid (3) during operation of said tank (1) and of the foam sucked in by said propeller (20) are formed, on one hand, by a first sleeve (22) disposed around the sheath (11) at the level of the first series of ports (17) and, on the other hand, by a second sleeve (23) disposed around said sheath (11) at the level of the second series of ports (18), the position of said sleeves (22, 23) relative to the corresponding ports (17, 18) being vertically adjustable.

5. Device according to any one of the preceding claims, **characterized in that** the means for effecting a static stirring by turbulence are formed, on one hand, by a vertical tube (31) disposed inside the sheath (11) and extending from the bottom of the tank (1) to below the anti-vortex means (24) and, on the other hand, by superimposed static obstacles (32) disposed inside said sheath (11) and fixed to said tube (31).

6. Device according to claim 5, **characterized in that** the static obstacles (32) define with the inner wall of the sheath (11) passages for the circulation of the liquid (3) during operation of said tank (1).

7. Device according to claims 5 and 6, **characterized in that** the static obstacles (32) are evenly spaced apart throughout the portion of the tube (31) disposed inside the sheath (11).

8. Device according to claims 5 and 6, **characterized in that** the static obstacles (32) are arranged on the tube (31), on one hand, below the anti-vortex means (24) and, on the other hand, in the lower part of the sheath (11).

9. Device according to claim 5, **characterized in that** the static stirring means employing turbulence are formed by static obstacles fixed to the inner wall of the sheath (11) and defining an axial passage for the circulation of the liquid (3) during operation of said tank (1).

10. Device according to claim 9, **characterized in that** the static obstacles are evenly spaced apart throughout the portion of the sheath (11) situated below the anti-vortex means (24).

11. Device according to claim 10, **characterized in that** the static obstacles are arranged inside the sheath (11), on one hand, below the anti-vortex means (24) and, on the other hand, in the lower part of the sheath (11).

12. Device according to any one of the claims 5 to 11, **characterized in that** the static obstacles are formed by cups (32) the concavity of which faces toward the bottom of the tank (1).

13. Device according to any one of the claims 5 to 11, **characterized in that** the static obstacles are formed by discs.

14. Device according to any one of the preceding claims, **characterized in that** the tank (1) is insulated.

15. Device according to any one of the preceding claims depending on claim 2, **characterized in that** the means for regulating the level of the liquid (3) in the funnel (14) during operation of said tank (1) are formed by an overflow pipe (25) disposed inside the funnel (14) in a position which is vertically adjustable.

16. Device according to any one of the preceding claims, **characterized in that** it comprises a second anti-vortex means (40) disposed inside the sheath (11) and above the main propeller (20), said second anti-vortex means (40) being formed by at least two vertical plates (40a) each extended upwardly by a stiffening rib (41) extending up to the upper end of said sheath (11).

17. Device according to any one of the preceding claims, **characterized in that** it comprises a liquid accelerator (45, 46, 47) inside the sheath (11).

18. Device according to any one of claims 2, 16 and 17, **characterized in that** the liquid accelerator is formed by, on one hand, a secondary propeller (45) disposed above the second anti-vortex means (40), connected to rotate with the shaft (19), and having a pitch oriented in the same direction as the pitch of the main propeller (20) and, on the other hand, an anti-vortex means (46) disposed above the secondary propeller (45) and inside a cylindrical stack (47) fixed to the reinforcing ribs (41), the secondary propeller (45), the anti-vortex means (46) and the stack (47) being situated between the two series of ports (17, 18).

19. Device according to any one of the claims 1 to 16, **characterized in that** it comprises means (50, 51, 52) for aerating the surface of the liquid during operation of said tank (1).

20. Device according to claims 2, 16 and 19, **characterized in that** the means for aerating the surface of the liquid during operation of said tank (1) comprise, on one hand, a secondary propeller (50) disposed above the second anti-vortex means (40), connected to rotate with the shaft (19) and having a pitch inverted relative to the pitch of the main propeller (20) and, on the other hand, an anti-vortex means (51) disposed above the secondary propeller (50) and inside a cylindrical stack (52) fixed to the reinforcing ribs (41), the secondary propeller (50) and the anti-vortex means (51) being situated between the two series of ports (17, 18) and the stack (52) extending substantially from the median part of the first series of ports (17) to almost the upper edge of the second series of ports (18), a cover (53) being mounted on the shaft (19) above the upper edge of the stack (52) and defines with said upper edge an adjustable passage (54).

21. Device according to claims 2 and 16, **characterized in that** it comprises a vortex pump (60) for the suction into the sheath (11) of the part of the liquid located close to the surface of this liquid and above the second anti-vortex means (40) and at the level of the first series of ports (17).

22. Device according to claim 21, **characterized in that** the vortex pump (60) comprises, on one hand, a circular plate (61) connected to rotate with the shaft (19) and provided on its upper side with radial and vertical blades (62) and, on the other hand, a stack (63) fixed to the upper edge of the blades (62) and extending from a position adjacent the upper end of the tank (1) substantially from the median part of the first series of ports (17) to said upper edge of the blades (62).

23. Device according to claim 22, **characterized in that** the stack (63) is conical and tapers toward the upper part of the tank (1).

24. Device according to claim 22, **characterized in that** the stack (63) is cylindrical.

25. Device according to any one of claims 22 to 24, **characterized in that** the blades (62) are planar or curved.

26. Device according to claims 2 and 16, **characterized in that** it comprises a single-passage propeller (70) for the suction of the liquid (3) into the sheath (11) during operation of said tank (1), disposed above the second anti-vortex means (40) and at the level of the first series of ports (17).

27. Device according to claim 26, **characterized in that** the single-passage propeller (70) comprises, on one hand, a circular plate (71) connected to rotate with the shaft (19) and disposed above the level that is to be reached by the liquid (3) in the tank (1) during operation of said tank (1) and, on the other hand, extending partially under said level, a vertical plate (72) forming a spiral and fixed to the lower face of said plate (71) around the shaft (19), said plate (72) defining a lateral inlet (73) for said liquid, a vertical passage (74) for the downward circulation of said liquid and a lower outlet (75) for the liquid.

28. Device according to claim 27, **characterized in that** the inner edge of the vertical plate (72) comprises a vertical deflector (76) which is inclined in the direction toward the center of the spiral.

29. Device according to claims 2 and 16, **characterized in that** it comprises a double-passage propeller (80) for the suction of the liquid (3) into the sheath (11) during operation of said tank (1), disposed above the second anti-vortex means (40) and at the level of the first series of ports (17).

30. Device according to claim 29, **characterized in that** the double-passage propeller (80) comprises, on one hand, a circular plate (81) connected to rotate with the shaft (19) and disposed above the level that is to be reached by the liquid (3) during operation of said tank (1) and, on the other hand, extending partially below said level, two vertical plates (82, 83) fixed to the lower face of said plate (81) and having the shape of two half-shells which are in opposed and offset relation to each other, said plates (82, 83) defining two lateral inlets (84, 85) for the liquid, two vertical passages (86, 87) for the downward circulation of the liquid and a lower outlet (88) for the liquid.

31. Device according to claim 30, **characterized in that** the edge of each plate (82, 83) situated in facing relation to the passage (86, 87) defined by the other plate includes a vertical deflector (89, 90).

32. Device according to any one of claims 26 to 31, **characterized in that** a pumping propeller (19) is fixed on the shaft (19) below the single passage propeller (70) or the double-passage propeller (80).

## Patentansprüche

1. Vorrichtung zum Rühren und Belüften einer Flüssigkeit sowie zur Schaumentfernung in einem Bottich (1) zum Behandeln der Flüssigkeit, die im Inneren des Bottichs (1) Folgendes umfasst:
- einen senkrechten Mantel (11), der sich über einen Teil der Höhe des Bottichs (1) erstreckt und in seinem oberen Teil mindestens eine erste Öffnung (17, 18) aufweist, die einen Eingangsdurchgang für die Flüssigkeit (3), die im Bottich (1) bei seiner Verwendung vorgesehen ist, einrichtet, und in der Nähe des Bodens dieses Bottichs mindestens eine zweite Öffnung, die für die Flüssigkeit (3) einen Ausgangsdurchgang (13) bildet, einrichtet,
- einen Trichter (14), der um den oberen Teil des Mantels (11) angeordnet und mit dem Mantel (11) verbunden ist, wobei der obere Rand dieses Trichters (14) mit der Wand des Bottichs (1) einen freien Raum (15) für den Durchgang der Flüssigkeit (3) bildet,
- eine Hauptschraube (20), die sich im Inneren des Mantels (11) unter dem Boden des Trichters (14) befindet,
- eine senkrechte motorisierte Welle (19), die diese Schraube (20), die sich einerseits bis über das obere Ende des Mantels (11) und andererseits bis unter den Boden des Trichters (14) erstreckt, in Drehung antreibt,
- ein Wirbelschutzorgan (24), das im Inneren des Mantels (11) unter der Schraube (20) angeordnet ist,
- Mittel (33) zum Einspritzen eines Gases unter Druck in den Mantel (11) unter dem Wirbelschutzorgan (24),
**dadurch gekennzeichnet, dass** sie außerdem nur statische Mittel zum Rühren (31, 32) durch Turbulenz der Flüssigkeit (3) im Inneren des Mantels (11) umfasst, wobei diese Mittel unter dem Wirbelschutzorgan (24) angeordnet sind.

2. Vorrichtung nach Anspruch 1, **dadurch gekennzeichnet, dass** sie Folgendes umfasst:
- eine erste Reihe von Fenstern (17), die im oberen Teil des Mantels (11) eingerichtet und so angeordnet sind, dass sie sich auf der Ebene der Flüssigkeit (3) befinden, die im Bottich (1) bei seiner Verwendung im Inneren des Trichters (14) vorgesehen ist,
- eine zweite Reihe Fenster (18), die im Mantel (11) direkt über dem Boden des Trichters (14) eingerichtet sind, und
- Mittel (25) zum Regeln des Niveaus der Flüssigkeit (3) im Trichter (14).

3. Vorrichtung nach Anspruch 2, **dadurch gekennzeichnet, dass** sie Mittel (22, 23) zum Regeln des Durchflusses der Flüssigkeit (3), die im Bottich (1) bei seiner Verwendung vorgesehen ist, und des Schaums, der von der Schraube (20) im Inneren des Mantels (11) angesaugt wird, umfasst.

4. Vorrichtung nach Anspruch 3, **dadurch gekennzeichnet, dass** die Mittel zum Regeln des Durchflusses der Flüssigkeit (3), die im Bottich (1) bei seiner Verwendung vorgesehen ist, und des von der Schraube (20) angesaugten Schaums einerseits aus einem ersten Stutzen (22), der um den Mantel (11) auf der Ebene der ersten Reihe von Fenstern (17) angeordnet ist, und andererseits aus einem zweiten Stutzen (23), der um den Mantel (11) auf der Ebene der zweiten Reihe von Fenstern (18) angeordnet ist, bestehen, wobei die Stellung dieser Stutzen (22, 23) zu den entsprechenden Fenstern (17, 18) senkrecht verstellbar ist.

5. Vorrichtung nach einem der vorhergehenden Ansprüche, **dadurch gekennzeichnet, dass** die statischen Mittel zum Rühren durch Turbulenz einerseits aus einem senkrechten Rohr (31), das im Inneren des Mantels (11) angeordnet ist und sich vom Boden des Bottichs (1) bis unter das Wirbelschutzorgan (24) erstreckt, und andererseits aus übereinander angeordneten statischen Hindernissen (32), die im Inneren des Mantels (11) angeordnet und am Rohr (31) befestigt sind, bestehen.

6. Vorrichtung nach Anspruch 5, **dadurch gekennzeichnet, dass** die statischen Hindernisse (32) mit der Innenwand des Mantels (11) Durchgänge für die Zirkulation der Flüssigkeit (3), die im Bottich (1) bei seiner Verwendunq vorgesehen ist, einrichten.

7. Vorrichtung nach den Ansprüchen 5 und 6, **dadurch gekennzeichnet, dass** die statischen Hindernisse (32) regelmäßig auf dem ganzen Teil des Rohrs (31), der im Mantel (11) angeordnet ist, verteilt sind.

8. Vorrichtung nach den Ansprüchen 5 und 6, **dadurch gekennzeichnet, dass** die statischen Hindernisse (32) auf dem Rohr (31) einerseits unter dem Wirbelschutzorgan (24) und andererseits im unteren Teil des Mantels (11) verteilt sind.

9. Vorrichtung nach Anspruch 5, **dadurch gekennzeichnet, dass** die statischen Mittel zum Rühren durch Turbulenz aus statischen Hindernissen bestehen, die auf der Innenwand des Mantels (11) befestigt sind und einen axialen Durchgang für die Zirkulation der Flüssigkeit (3), die im Bottich (1) bei seiner Verwendung vorgesehen ist, einrichten.

10. Vorrichtung nach Anspruch 9, **dadurch gekennzeichnet, dass** die statischen Hindernisse regelmäßig auf den ganzen Teil des Mantels (11) verteilt sind, der unter dem Wirbelschutzorgan (24) liegt.

11. Vorrichtung nach Anspruch 10, **dadurch gekennzeichnet, dass** die statischen Hindernisse im Inneren des Mantels (11) einerseits unter dem Wirbelschutzorgan (24) und andererseits im unteren Teil des Mantels (11) verteilt sind.

12. Vorrichtung nach einem der Ansprüche 5 bis 11, **dadurch gekennzeichnet, dass** die statischen Hindernisse aus Schalen (32) bestehen, deren Konkavität zum Boden des Bottichs (1) gerichtet ist.

13. Vorrichtung nach einem der Ansprüche 5 bis 11, **dadurch gekennzeichnet, dass** die statischen Hindernisse aus Scheiben bestehen.

14. Vorrichtung nach einem der vorhergehenden Ansprüche, **dadurch gekennzeichnet, dass** der Bottich (1) isoliert ist.

15. Vorrichtung nach einem der vorhergehenden Ansprüche, wenn dieser von Anspruch 2 abhängt, **dadurch gekennzeichnet, dass** die Mittel zum Regeln des Niveaus im Trichter (14) der Flüssigkeit (3), die im Bottich (1) bei seiner Verwendung vorgesehen ist, aus einem Überlaufschlauch (25) bestehen, der im Inneren des Trichters (14) angeordnet und dessen Stellung senkrecht verstellbar ist.

16. Vorrichtung nach einem der vorhergehenden Ansprüche, **dadurch gekennzeichnet, dass** sie ein zweites Wirbelschutzorgan (40) umfasst, das im Inneren des Mantels (11) und über der Hauptschraube (20) angeordnet ist, wobei dieses zweite Wirbelschutzorgan (40) aus mindestens zwei senkrechten Platten (40a) besteht, die jeweils nach oben durch eine Verstärkungsrippe (41), die sich bis zum oberen Ende des Mantels (11) erstreckt, verlängert sind.

17. Vorrichtung nach einem der vorhergehenden Ansprüche, **dadurch gekennzeichnet, dass** sie einen Flüssigkeitsbeschleuniger (45, 46, 47) im Inneren des Mantels (11) umfasst.

18. Vorrichtung nach einem der Ansprüche 2, 16 und 17, **dadurch gekennzeichnet, dass** der Flüssigkeitsbeschleuniger einerseits aus einer Nebenschraube (45) besteht, die über dem zweiten Wirbelschutzorgan (40) angeordnet in Drehung fest mit der Welle (19) verbunden ist und einen Schraubengang in die gleiche Richtung wie der Schraubengang der Hauptschraube (20) hat, und andererseits aus einem Wirbelschutzorgan (46), das über der Nebenschraube (45) und im Inneren eines zylindrischen Schachts (47) angeordnet ist, der auf den Verstärkungsrippen (41) befestigt ist, besteht, wobei die Nebenschraube (45), das Wirbelschutzorgan (46) und der Schacht (47) zwischen den beiden Reihen von Fenstern (17, 18) liegen.

19. Vorrichtung nach einem der Ansprüche 1 bis 16, **dadurch gekennzeichnet, dass** sie Mittel (50, 51, 52) zum Belüften der Oberfläche der Flüssigkeit umfasst, die im Bottich (1) bei seiner Verwendung vorgesehen ist.

20. Vorrichtung nach den Ansprüchen 2, 16 und 19, **dadurch gekennzeichnet, dass** die Mittel zur Belüftung der Oberfläche der Flüssigkeit, die im Bottich (1) bei seiner Verwendung vorgesehen sind, einerseits aus einer Nebenschraube (50) bestehen, die über dem zweiten Wirbelschutzorgan (40) in Drehung fest mit der Welle (19) verbunden angeordnet ist und einen Schraubengang hat, der zum Schraubengang der Hauptschraube (20) entgegengesetzt ist, und andererseits aus einem Wirbelschutzorgan (51), das über der Nebenschraube (50) und im Inneren eines zylindrischen Schachts (52) angeordnet ist, der auf den Verstärkungsrippen (41) befestigt ist, besteht, wobei die Nebenschraube (50) und das Wirbelschutzorgan (51) zwischen den zwei Reihen von Fenstern (17, 18) und dem Schacht (52), der sich im Wesentlichen vom mittleren Teil der ersten Reihe von Fenstern (17) bis fast zum oberen Rand der zweiten Reihe von Fenstern (18) erstreckt, liegen, wobei ein Deckel (53) auf der Welle (19) über dem oberen Rand des Schachts (52) installiert ist und mit dem oberen Rand einen verstellbaren Durchgang (54) bildet.

21. Vorrichtung nach den Ansprüchen 2 und 16, **dadurch gekennzeichnet, dass** sie eine Wirbelpumpe (60), die oberhalb des zweiten Wirbelschutzorgans (40) und in Höhe der ersten Reihe von Fenstern (17) angeordnet ist zum Ansaugen desjenigen Teils der im Bottich (1) bei seiner Verwendung vorgesehenen Flüssigkeit, der sich in der Nähe der Oberfläche dieser Flüssigkeit befindet, in das Innere des Mantels (11) umfasst.

22. Vorrichtung nach Anspruch 21, **dadurch gekennzeichnet, dass** die Wirbelpumpe (60) einerseits eine kreisförmige Platte (61) umfasst, die fest in Drehung mit der Welle (19) verbunden und auf ihrer oberen Seite mit radialen und senkrechten Schaufeln (62) versehen ist, und andererseits einen Schacht (63) umfasst, der auf dem oberen Rand der Schaufeln (62) befestigt ist und sich zum oberen Teil des Bottichs (1) im Wesentlichen vom mittleren Teil der ersten Reihe von Fenstern (17) bis zum oberen Rand der Schaufeln (62) hin erstreckt.

23. Vorrichtung nach Anspruch 22, **dadurch gekennzeichnet, dass** der Schacht (63) kegelstumpfförmig ist, und dass seine Konizität zum oberen Teil des Bottichs (1) gerichtet ist.

24. Vorrichtung nach Anspruch 22, **dadurch gekennzeichnet, dass** der Schacht (63) zylindrisch ist.

25. Vorrichtung nach einem der Ansprüche 22 bis 24, **dadurch gekennzeichnet, dass** die Schaufeln (62) gerade oder gebogen sind.

26. Vorrichtung nach den Ansprüchen 2 und 16, **dadurch gekennzeichnet, dass** sie eine Einkanalschraube (70), die über dem zweiten Wirbelschutzorgan (40) und auf der Ebene der ersten Reihe von Fenstern (17) angeordnet ist, zum Ansaugen der im Bottich (1) bei seiner Verwendung vorgesehenen Flüssigkeit (3) in das Innere des Mantels (11) umfasst.

27. Vorrichtung nach Anspruch 26, **dadurch gekennzeichnet, dass** die Einkanalschraube (70) einerseits eine kreisförmige Platte (71) umfasst, die fest in Drehung mit der Welle (19) verbunden und über dem Niveau angeordnet ist, das die Flüssigkeit (3), die im Bottich (1) bei seiner Verwendung vorgesehen ist, erreichen muss, und andererseits eine senkrechte Platte (72) umfasst, die sich zum Teil unter dem Niveau erstreckt, die eine Spirale bildet und auf der unteren Seite der Platte (71) um die Welle (19) befestigt ist, wobei die Platte (72) einen seitlichen Eingang (73) der Flüssigkeit, einen senkrechten Kanal (74) zur Zirkulation der Flüssigkeit von oben nach unten und einen unteren Ausgang (75) der Flüssigkeit abgrenzt.

28. Vorrichtung nach Anspruch 27, **dadurch gekennzeichnet, dass** der innere Rand der senkrechten Platte (72) eine senkrechte Ablenkvorrichtung (76) umfasst, die in Richtung der Mitte der Spirale geneigt ist.

29. Vorrichtung nach den Ansprüchen 2 und 16, **dadurch gekennzeichnet, dass** sie eine Zweikanalschraube (80) umfasst, die über dem zweiten Wirbelschutzorgan (40) und auf der Ebene der ersten Reihe von Fenstern (17) angeordnet ist, zum Ansaugen der im Bottich (1) bei seiner Verwendung vorgesehenen Flüssigkeit (3) in das Innere des Mantels (11).

30. Vorrichtung nach Anspruch 29, **dadurch gekennzeichnet, dass** die Zweikanalschraube (80) einerseits eine kreisförmige Platte (81) umfasst, die fest in Drehung mit der Welle (19) verbunden und über dem Niveau angeordnet ist, das die Flüssigkeit (3), die im Bottich (1) bei seiner Verwendung vorgesehen ist, erreichen muss und, andererseits zwei senkrechte Platten (82, 83) umfasst, die sich zum Teil unter dem Niveau erstrecken und auf der unteren Seite der Platte (81) befestigt sind und die Form zweier einander gegenüber liegender Halbschalen, die zueinander versetzt sind, haben, wobei die Platten (82, 83) zwei seitliche Eingänge (84, 85) der Flüssigkeit, zwei senkrechte Kanäle (86, 87) zur Zirkulation dieser Flüssigkeit von oben nach unten und einen unteren Ausgang (88) der Flüssigkeit bilden.

31. Vorrichtung nach Anspruch 30, **dadurch gekennzeichnet, dass** der Rand jeder Platte (82, 83), der dem Kanal (86, 87), der von der anderen Platte abgegrenzt wird, gegenüber liegt, eine senkrechte Ablenkvorrichtung (89, 90) umfasst.

32. Vorrichtung nach einem der Ansprüche 26 bis 31, **dadurch gekennzeichnet, dass** eine Pumpschraube auf der Welle (19) unter der Einkanalschraube (70) oder der Zweikanalschraube (80) befestigt ist.
